# EUROPEAN PATENT APPLICATION

(11) **EP 1 266 654 A1**
(43) Date of publication of application: **18.12.2002**
(21) Application number: 02253795.5
(22) Date of filing: 30.05.2002
(51) Int. Cl.: A61K 9/14, A61K 9/16, A61K 47/12, A61K 31/4422

(54) **Stabilised amlodipine maleate formulations**

(30) Priority: 15.06.2001 GB 0114709
(71) Applicant: Pfizer Limited, Sandwich, Kent CT13 9NJ (GB); PFIZER INC., New York, N.Y. 10017 (US)
(72) Inventor: Bilotte, Anne, Pfizer Global Res. & Development, CA 92037 (US); Pavitt, Sharon, Pfizer Global Res. & Development, Sandwich, Kent CT13 9NJ (GB); Pettman, Alan J., Pfizer Global Res. & Development, Sandwich, Kent CT13 9NJ (GB); Smith, Zena E.F., Pfizer Global Res. & Development, Sandwich, Kent CT13 9NJ (GB)
(74) Representative: Wood, David John

(57) **Abstract**

The present invention is concerned with means for stabilising amlodipine maleate to prevent the formation of unwanted Michael adduct and with stabilised pharmaceutical compositions obtained thereby.

## Description

The present invention is concerned with the stabilisation of amlodipine maleate and with stabilised pharmaceutical compositions obtained thereby.

The preparation of amlodipine and many of its salts is described in European Patent No. 0089167. The maleate is typically prepared by treating amlodipine free base with maleic acid in a suitable solvent, such as ethyl acetate (Examples 11, 23 and 24), ethanol (Example 12), or industrial methylated spirit (Example 22), and collecting, washing and drying the resulting precipitate. Under such conditions, the free base, in addition to forming the desired salt, is prone to undergo a Michael addition reaction with the maleic acid which results in the formation of a small amount (<0.5% by weight) of an unwanted adduct, N-(2-{[4-(2-chlorophenyl)-3-(ethoxycarbonyl)-5-(methoxycarbonyl)-6-methyl-1,4-dihydro-2-pyridyl]methoxy}ethyl)aspartic acid:

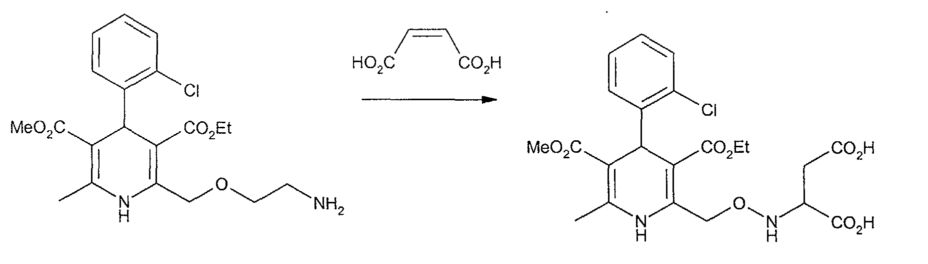

In addition to the small amount of adduct formed during salt formation, any unreacted free base and maleic acid remaining in the bulk substance will, under suitable conditions, particularly the presence of moisture, continue to react to give more of the unwanted adduct. Further adduct formation is particularly noticeable during formulation of the bulk substance and can continue throughout the life of the stored product.

For example, when bulk substance is formulated as immediate release hard gelatine capsules, the amount of adduct in the capsules increases to 1% by weight after 1 year's storage, while conventional immediate release tablets contain 2% by weight of the adduct (with concomitant discoloration) after only 12 weeks at 37°C. Difficulties in formulating tablets due to adhesion of bulk substance to the tableting equipment have also been attributed to the presence of Michael adduct.

Thus formation of the adduct has serious implications both for formulating the bulk substance, especially as tablets, and for obtaining satisfactory regulatory approval of the resulting products, particularly with regard to permitted shelf life. The involvement of moisture in the formation of the adduct requires special packaging if even a minimum acceptable shelf life is to be achieved.

We have now found that it is possible to stabilise compositions containing amlodipine maleate by adding an appropriate amount of certain acids to the bulk substance prior to formulation. The presence of these acids unexpectedly prevents the formation of further Michael adduct both during formulation and throughout the life of the product, thereby providing a stable pharmaceutical composition for the purposes of obtaining regulatory approval with associated cost savings in packaging.

According to the present invention, therefore, there is provided a pharmaceutical composition comprising amlodipine maleate and a pharmaceutically acceptable carrier, characterised in that said formulation additionally comprises an acid having a pK₁ of greater than 1.5 which is present in an amount sufficient to prevent the formation of N-(2-{[4-(2-chlorophenyl)-3-(ethoxycarbonyl)-5-(methoxycarbonyl)-6-methyl-1,4-dihydro-2-pyridyl]methoxy}ethyl)aspartic acid. In a preferred embodiment, said acid is present in an amount of up to 3% by weight.

Preferred acids for the purposes of the invention are maleic acid (pK₁ 1.9), phosphoric acid (pK₁ 2.12), ascorbic acid (pK₁ 4.17) and sorbic acid (pK₁ 4.76).

It has been found that the stabilising effect of the added acid is enhanced by the selection of an appropriate carrier, preferably mannitol, a cellulosic material, or a starch (or a mixture thereof), which carrier is typically present in an amount of at least 90% by weight.

Therefore according to another aspect of the invention, there is provided a pharmaceutical composition comprising amlodipine maleate and a pharmaceutically acceptable carrier, characterised in that said formulation comprises (i) an acid having a pK₁ of greater than 1.5 which is present in an amount sufficient to prevent the formation of N-(2-{[4-(2-chlorophenyl)-3-(ethoxycarbonyl)-5-(methoxycarbonyl)-6-methyl-1,4-dihydro-2-pyridyl]methoxy}ethyl)aspartic acid and (ii) said carrier is mannitol, a cellulosic material, or a starch (or a mixture thereof).

Said acid is preferably present in an amount of up to 3% by weight. Particularly preferred cellulosic materials or the purposes of the invention are Elcema® and ethylcellulose, particularly preferred starches are maize starch and Starch 1500®.

It has been found that the stabilising effect of the added acid when used in conjunction with an appropriate carrier can be further enhanced by the inclusion in the formulation of a disintegrant, which disintegrant is typically present in an amount of up to 10% by weight.

Therefore according to yet another aspect of the invention, there is provided a pharmaceutical composition comprising amlodipine maleate and a pharmaceutically acceptable carrier, characterised in that said formulation comprises (i) an acid having a pK₁ of greater than 1.5 which is present in an amount sufficient to prevent the formation of N-(2-{[4-(2-chlorophenyl)-3-(ethoxycarbonyl)-5-(methoxycarbonyl)-6-methyl-1,4-dihydro-2-pyridyl]methoxy}ethyl)aspartic acid, (ii) said carrier is mannitol, a cellulosic material, or a starch (or a mixture thereof) and (iii) a disintegrant.

Said acid is preferably present in an amount of up to 3% by weight. Particularly preferred cellulosic materials are Elcema® and ethylcellulose, particularly preferred starches are maize starch and Starch 1500®. A preferred disintegrant for the purposes of the invention is Ac-Di-Sol®.

It has been found that the stabilising effect of the added acid when used in conjunction with an appropriate carrier and a disintegrant can be further enhanced by the inclusion in the formulation of a lubricant, which lubricant is typically present in an amount of up to 1% by weight.

Therefore according to yet another aspect of the invention, there is provided a pharmaceutical composition comprising amlodipine maleate and a pharmaceutically acceptable carrier, characterised in that said formulation comprises (i) an acid having a pK₁ of greater than 1.5 which is present in an amount sufficient to prevent the formation of N-(2-{[4-(2-chlorophenyl)-3-(ethoxycarbonyl)-5-(methoxycarbonyl)-6-methyl-1,4-dihydro-2-pyridyl]methoxy}ethyl)aspartic acid, (ii) said carrier is mannitol, a cellulosic material, or a starch (or a mixture thereof), (iii) a disintegrant and (iv) a lubricant.

Said acid is preferably present in an amount of up to 3% by weight. Particularly preferred cellulosic materials are Elcema® and ethylcellulose, particularly preferred starches are maize starch and Starch 1500®. A preferred disintegrant is Ac-Di-Sol®. A preferred lubricant for the purposes of the invention is magnesium stearate.

It has been found that a significant reduction in the formation of Michael adduct can also be achieved (in the absence of acid) by the selection of an appropriate particle size range for the dry powder blend, typically wherein more than 10% of the particles have a size greater than 10 microns.

Therefore according to another aspect of the invention, there is provided a pharmaceutical composition comprising amlodipine maleate and a pharmaceutically acceptable carrier, characterised in that said composition additionally comprises particles of which more than 10% have a size greater than 10 microns.

It has been found that a significant reduction in the formation of Michael adduct can also be achieved (in the absence of acid) by the choice of an appropriate carrier, particularly one having a particle shape which is averse to the entry of water. Elcema®, for example, which has long, lamellar particles which are smooth and flat in shape with a non-porous surface, appears to reduce adduct formation by limiting the access of water. Ethylcellulose, mannitol and Starch 1500® are also particularly suitable for this purpose.

Therefore according to another aspect of the invention, there is provided a pharmaceutical composition comprising amlodipine maleate and a pharmaceutically acceptable carrier, characterised in that said carrier is Elcema®, ethylcellulose, mannitol, or Starch 1500®.

Stabilised dry powder blends in accordance with the invention may be formulated, for example, by compressing into tablets or filling into capsules (with or without prior conversion to a granulated powder by means such as described in Examples 18 and 19) to give a pharmaceutical composition having excellent shelf life.

Tablets according to the invention typically contain from 0.1 mg to 20mg of amlodipine maleate and have a fill weight of between 50mg and 500mg. Suitable carriers include microcrystalline cellulose, lactose, sodium citrate, calcium carbonate, dibasic calcium phosphate, glycine and starch (preferably corn, potato or tapioca starch), disintegrants such as sodium starch glycollate, croscarmellose sodium and certain complex silicates, and granulation binders such as polyvinylpyrrolidine, hydroxypropylmethylcellulose (HPMC), hydroxypropylcellulose (HPC), sucrose, gelatin and acacia. Lubricants such as magnesium stearate, stearic acid, glyceryl behenate and talc may be included and the tablet core coated with an appropriate overcoat.

Capsules according to the invention are typically of gelatin or HPMC. Preferred excipients include lactose, starch, a cellulose, milk sugar and high MW polyethylene glycols.

The invention is illustrated by the following examples:

### EXAMPLE 1 (COMPARATIVE)

A binary dry powder blend comprising amlodipine maleate (1.67%), Avicel PH101® (73.5%), maize starch (24.3%) and magnesium stearate (0.5%)

### EXAMPLE 2

A binary dry powder blend comprising amlodipine maleate (1.67%), mannitol (73.6%), maize starch (24.3%) and magnesium stearate (0.5%)

### EXAMPLE 3

A binary dry powder blend comprising amlodipine maleate (1.67%), mannitol (73.6%), maize starch (24.3%) and magnesium stearate (0.5%), wherein more than 10% of the particles have a size greater than 10 microns.

### EXAMPLE 4

A dry powder blend comprising amlodipine maleate (1.67%), mannitol (73.1%), maize starch (23.8%), magnesium stearate (0.5%) and maleic acid (1%)

### EXAMPLE 5

A dry powder blend comprising amlodipine maleate (1.67%), mannitol (72.8%), maize starch (23.5%), magnesium stearate (0.5%) and ascorbic acid (1.51%)

### EXAMPLE 6

A dry powder blend comprising amlodipine maleate (1.67%), mannitol (73%), maize starch (21%), magnesium stearate (0.5%) and ascorbic acid (3%)

### EXAMPLE 7

A dry powder blend comprising amlodipine maleate (1.67%), mannitol (73%), maize starch (24%), magnesium stearate (0.5%) and sorbic acid (0.96%)

### EXAMPLE 8 (COMPARATIVE)

A binary dry powder blend comprising amlodipine maleate (1.67%) and Ac-Di-Sol®

### EXAMPLE 9 (COMPARATIVE)

A binary dry powder blend comprising amlodipine maleate (1.67%) and Avicel PH101®

### EXAMPLE 10 (COMPARATIVE)

A binary dry powder blend comprising amlodipine maleate (1.67%) and calcium carbonate

### EXAMPLE 11 (COMPARATIVE)

A binary dry powder blend comprising amlodipine maleate (1.67%) and Di-Tab®

### EXAMPLE 12 (COMPARATIVE)

A binary dry powder blend comprising amlodipine maleate (1.67%) and ExploTab®

### EXAMPLE 13 (COMPARATIVE)

A binary dry powder blend comprising amlodipine maleate (1.67%) and magnesium stearate

### EXAMPLE 14

A binary dry powder blend comprising amlodipine maleate (1.67%) and Elcema®

### EXAMPLE 15

A binary dry powder blend comprising amlodipine maleate (1.67%) and ethylcellulose

### EXAMPLE 16

A binary dry powder blend comprising amlodipine maleate (1.67%) and mannitol

### EXAMPLE 17

A binary dry powder blend comprising amlodipine maleate (1.67%) and Starch 1500®

The dry powder blends of Examples 1-17 were obtained by
(1) weighing the amlodipine maleate into a suitable container;
(2) weighing the excipients into a separate container and blending with a Turbula mixer for 10 minutes;
(3) adding about 8% by weight of the excipient preblend to the amlodipine maleate, blending with the Turbula mixer for 10 minutes, then screening through a 500µm mesh sieve;
(4) adding about 40% by weight of the excipient preblend to the amlodipine maleate mix, blending with the Turbula mixer for 10 minutes, then screening through a 500µm mesh sieve; and
(5) adding remaining 52% by weight of excipient preblend to amlodipine maleate mix, blending with the Turbula mixer for 10 minutes, screening through a 500µm mesh sieve and finally blending with the Turbula mixer for 10 minutes.

The dry powder blends of Examples 1-17 were checked for homogeneity by HPLC.

### RESULTS FOR EXAMPLES 1-17

After 6 weeks at 40°C/75% relative humidity, the stabilised dry powder blends of Examples 1-17 were analysed for Michael adduct by HPLC. The results were as follows:

| EXAMPLE | % MICHAEL ADDUCT BY WEIGHT |
|---|---|
| 1 (comparative) | 0.4 |
| 2 | 0.2 |
| 3 | 0.3 |
| 4 | 0.06 |
| 5 | 0.1 |
| 6 | 0.1 |
| 7 | 0.2 |
| 8 (comparative) | 0.1 |
| 9 (comparative) | 0.9 |
| 10 (comparative) | 1.0 |
| 11 (comparative) | 2.2 |
| 12 (comparative) | 5.6 |
| 13 (comparative) | 0.3 |
| 14 | 0.3 |
| 15 | 0.2 |
| 16 | 0.09 |
| 17 | 0.2 |

After 12 weeks at 40°C/75% relative humidity, the stabilised dry powder blends of Examples 1-5, 9, 14, 16 and 17 were again analysed for Michael adduct by HPLC. The results were as follows:

| EXAMPLE | % MICHAEL ADDUCT BY WEIGHT |
|---|---|
| 1 (comparative) | 1.0 |
| 2 | 0.4 |
| 3 | 0.4 |
| 4 | 0.09 |
| 5 | 0.1 |
| 9 (comparative) | 1.0 |
| 14 | 0.3 |
| 16 | 0.1 |
| 17 | 0.2 |

### EXAMPLE 18 (COMPARATIVE)

A dry powder blend comprising amlodipine maleate (1.67%), Avicel PH101® (75%), Starch 1500® (13%) and AcDiSol® (10%) was obtained using a blend/screen process analogous to that described for Examples 1-17 and converted to a granulated powder by the following additional steps:
(6) preparing an aqueous solution of EDTA (0.05%);
(7) adding solution to dry powder blend by 'wet massing' procedure using a high shear mixer - additional water added so that when compressed the blend broke cleanly;
(8) forcing blend through 1mm sieve to give a granulated powder; and
(9) drying the resulting powder on a tray overnight at 50°C.

The granulated powder of Example 18 was analysed by HPLC to determine initial amlodipine maleate concentration.

### EXAMPLE 19

A dry powder blend comprising amlodipine maleate (1.67%), Avicel PH101® (75%), Starch 1500® (13%) and AcDiSol® (10%) was obtained using a blend/screen process analogous to that described for Examples 1-17 and converted to a granulated powder by the following additional steps:
(6) preparing an aqueous solution of EDTA (0.05%) and phosphoric acid (0.01%);
(7) adding solution to dry powder blend by 'wet massing' procedure using a high shear mixer - additional water added so that when compressed the blend broke cleanly;
(8) forcing blend through 1mm sieve to give a granulated powder; and
(9) drying the resulting powder on a tray overnight at 50°C.

The granulated powder of Example 19 was analysed by HPLC to determine initial amlodipine maleate concentration.

### RESULTS FOR EXAMPLES 18 AND 19

After 6 weeks at 40°C/75% relative humidity, the granulated powders of Examples 18 and 19 were analysed for Michael adduct by HPLC. The results were as follows:

| EXAMPLE | % MICHAEL ADDUCT BY WEIGHT |
|---|---|
| 18 (comparative) | 2.3 |
| 19 | 0.3 |

## Claims

1. A pharmaceutical composition comprising amlodipine maleate and a pharmaceutically acceptable carrier, **characterised in that** said composition additionally comprises an acid having a pK₁ of greater than 1.5 which is present in an amount sufficient to prevent the formation of N-(2-{[4-(2-chlorophenyl)-3-(ethoxycarbonyl)-5-(methoxycarbonyl)-6-methyl-1,4-dihydro-2-pyridyl]methoxy}ethyl)aspartic acid.

2. A composition according to Claim 1, wherein said acid is present in an amount of up to 3% by weight.

3. A composition according to Claim 1 or 2, wherein said acid is maleic acid, phosphoric acid, ascorbic acid, or sorbic acid.

4. A composition according to any of Claims 1 to 3, wherein the carrier is mannitol.

5. A composition according to any of Claims 1 to 3, wherein the carrier is a cellulosic material.

6. A composition according to Claim 5, wherein the cellulosic material is Elcema® or ethylcellulose.

7. A composition according to any of Claims 1 to 3, wherein the carrier is a starch.

8. A composition according to Claim 7, wherein the carrier is maize starch or Starch 1500®.

9. A composition according to any of Claims 1 to 8, wherein the carrier is present in an amount of at least 90% by weight.

10. A composition according to any of Claims 1 to 9, which additionally comprises a disintegrant.

11. A composition according to Claim 10, wherein said disintegrant is present in an amount of up to 10% by weight.

12. A composition according to Claims 10 and 11, wherein said disintegrant is AcDiSol®.

13. A composition according to any of Claims 1 to 12 which additionally comprises a lubricant.

14. A composition according to Claim 13, wherein said lubricant is present in an amount of up to 1% by weight.

15. A composition according to Claims 12 and 13, wherein said lubricant is magnesium stearate.

16. A composition according to any of Claims 1 to 15 which is in the form of a tablet.

17. A composition according to any of Claims 1 to 15 which is in the form of a capsule.

18. A pharmaceutical composition comprising amlodipine maleate and a pharmaceutically acceptable carrier, **characterised in that** more than 10% of the particles therein have a size greater than 10 microns.

19. A pharmaceutical composition comprising amlodipine maleate and a pharmaceutically acceptable carrier, **characterised in that** said carrier is Elcema®, ethylcellulose, mannitol, or Starch 1500®.
